# EUROPEAN PATENT APPLICATION

(11) **EP 3 950 052 A1**
(43) Date of publication of application: **09.02.2022**
(21) Application number: 20785317.7
(22) Date of filing: 01.04.2020
(51) Int. Cl.: A61N 1/375, A61N 1/05

(54) **LEADLESS PACEMAKER AND LEADLESS PACEMAKER SYSTEM**

(30) Priority: 02.04.2019 CN 201910262708
(71) Applicant: MicroPort Soaring CRM (Shanghai) Co., Ltd., Shanghai 201203 (CN)
(72) Inventor: WANG, Li, Shanghai 201203 (CN); CHENG, Zhijun, Shanghai 201203 (CN)
(74) Representative: Lavoix
(86) International application number: PCT/CN2020/082685
(87) International publication number: WO 2020/200223

(57) **Abstract**

A leadless pacemaker and a leadless pacemaker system are disclosed. A housing of a pacemaker body of the leadless pacemaker is designed as a curved tubular structure and can be deployed at a site with a limited space in a curved configuration, which allows the curved surface structure to fit more closely myocardial or vascular tissue around the site, resulting in improved pacing or sensing performance and in applicability to a substantially enlarged scope of patients who may benefit from cardiac pacing.

## Description

### TECHNICAL FIELD

The present invention relates to the field of medical instruments and, in particular, to a leadless pacemaker and a leadless pacemaker system.

### BACKGROUND

Since the advent in 1958, cardiac pacemakers have become a first-line treatment for bradycardia arrhythmia. With the advancements and innovations over the past more than half century, pacemakers have gradually evolved from the initial form of single leads for open chest implantation and ventricular pacing to the recent form of 2-3 leads for atrioventricular physiological pacing or even biventricular synchronous pacing. However, lead-related complications such as lead dislodgement, thrombosis, tricuspid regurgitation and infection have always been a handicap that not only interrupts the normal operation of pacemakers but also seriously threatens patients' health and lives and degrades their quality of life. Moreover, the removal of such leads is associated with certain difficulties and risks and must performed in large electrophysiology centers by dexterous surgeons. In order to overcome these problems arising from the use of leads, "leadless" cardiac pacemakers have become a new focus of interest in the field of arrhythmia treatment.

Conventional leadless cardiac pacemakers typically include a fixation mechanism that is separate from a pacing electrode and has a diameter equal to or less than an outer diameter of the pacemaker. The fixation mechanism is designed to penetrate tissue when rotated two or more revolutions, thus bringing the pacing electrode into contact with the tissue and achieving its fixation. There are also some leadless pacemakers including a circular ring-shaped structure disposed at a proximal end of the leadless pacemaker, which can tether the leadless pacemaker within the right ventricle upon the failure of the distal fixation mechanism, preventing its entry into the blood circulation and consequent danger to the patient.

These leadless pacemakers are associated with the following drawbacks:

They have a straight elongate body and their fixation relies on the screwing of a helical electrode arranged at the leading end into the relatively thick apical myocardial tissue. With this design, they must be fixed in a perpendicular orientation and thus have a high profile, making them difficult to be deployed at sites with limited spaces available for their accommodation, such as blood vessel walls or the interventricular septum. In other words, since the helical electrode extends in parallelism and coaxiality with the pacemaker body, these leadless pacemakers require a deployment space that can accommodate their length. Therefore, they are not suitable to be fixed at sites with limited spaces, such as blood vessel walls or the interventricular septum, in spite of the fact that these sites might be ideal for the pacing purposes.

Therefore, pacing performance of the conventional leadless pacemakers is suboptimal because they cannot be deployed at ideal pacing sites with limited spaces.

### SUMMARY OF THE INVENTION

It is an objective of the present invention to provide a leadless pacemaker and a leadless pacemaker system, which are applicable to a substantially enlarged scope of patients who may benefit from cardiac pacing.

To this end, the provided leadless pacemaker includes:
a pacemaker body including a curved tubular housing;
a fixation mechanism disposed on an outer wall of the curved tubular housing;
a sensing electrode disposed outside the curved tubular housing; and
a pacing electrode disposed outside the curved tubular housing so as to be spaced apart from the sensing electrode.

Optionally, the sensing electrode may be disposed on the curved tubular housing so as to extend in a circumferential direction thereof to surround the outer wall of the curved tubular housing, or on the fixation mechanism.

Optionally, the pacing electrode may be disposed on the curved tubular housing so as to extend in a circumferential direction thereof to surround the outer wall of the curved tubular housing, or on the fixation mechanism.

Optionally, when the leadless pacemaker is delivered to a target pacing site, the pacing and sensing electrodes may be positionable at the same height.

Optionally, the fixation mechanism may be able to assume an unfolded or folded configuration with respect to the curved tubular housing.

Optionally, the fixation mechanism may include at least one sharp-pointed hook having a trailing end secured to the curved tubular housing and a leading end extending toward a leading end of the curved tubular housing, the leading end of the sharp-pointed hook spaced from an outer circumference of the curved tubular housing by a gap when in a rest state, thereby defining the unfolded configuration with respect to the curved tubular housing, the leading end of the sharp-pointed hook retracted onto the curved tubular housing under the action of an external force applied thereto, thereby defining the folded configuration with respect to the curved tubular housing.

Optionally, a hook receiving recess may be provided in the outer wall of the curved tubular housing and configured to receive the at least one sharp-pointed hook so that the trailing end of each sharp-pointed hook is secured in the hook receiving recess and that each sharp-pointed hook is received, under the action of an external force applied to its leading end, in the hook receiving recess along its entire length.

Optionally, each sharp-pointed hook may be provided in the form of a wire, a rod or a tab.

Optionally, the sharp-pointed hook may be arranged on a convexly curved outer surface portion of the curved tubular housing.

Optionally, the pacing or sensing electrode may be disposed on one of the sharp-pointed hook(s), wherein both the pacing and sensing electrodes are electrically connected to an electronic component inside the curved tubular housing.

Optionally, the curved tubular housing may include a first curved tubular segment and a second curved tubular segment, the second curved tubular segment having a leading end coupled to a trailing end of the first curved tubular segment, the second curved tubular segment having a trailing end elevated over the first curved tubular segment, the first curved tubular segment having a leading end forming a leading end of the curved tubular housing, the trailing end of the second curved tubular segment forming a trailing end of the curved tubular housing.

Optionally, both the first and second curved tubular segments may be arc-shaped or wave-shaped and a length of the first curved tubular segment may be greater than a length of the second curved tubular segment.

Optionally, the curved tubular housing may have a cross section along its radial direction, which is polygonal or defined by a closed wavy line.

Optionally, the curved tubular housing may be made of an elastically deformable material.

Optionally, the curved tubular housing may be provided at a trailing end thereof with a coupling mechanism configured for coupling to a delivery member for delivering the leadless pacemaker.

The leadless pacemaker system is based on the same inventive concept and includes:
the leadless pacemaker of the present invention as defined above; and
a delivery member detachably coupled to the trailing end of the pacemaker body of the leadless pacemaker, the delivery member configured to deliver the leadless pacemaker to a target pacing site.

Optionally, the delivery member may include a delivery sheath and a rotating sheath receivable in the delivery sheath, the delivery sheath axially movable and circumferentially rotatable relative to the rotating sheath, the rotating sheath having a leading end configured for detachable coupling to the trailing end of the pacemaker body of the leadless pacemaker.

Optionally, the delivery sheath may be a curved sheath with a degree of curvature that is adjustable.

The present invention has the following advantages over the prior art:
1. Since the housing of the pacemaker body of the leadless pacemaker is designed as a curved structure, it can be deployed at a site with a limited space (dimensioned less than the length of the curved tubular housing) in a curved configuration, making it applicable to patients with ideal pacing sites with limited spaces. Therefore, the leadless pacemaker is applicable to a substantially enlarged scope of patients who may benefit from cardiac pacing.
2. Since the housing of the pacemaker body of the leadless pacemaker is designed as a curved structure, the leadless pacemaker can be deployed at a site associated with a curved surface geometry in such a manner that it more closely fits surrounding myocardial or vascular tissue at the site that defines the curved surface geometry, resulting in improved pacing or sensing performance and in applicability to a substantially enlarged scope of patients who may benefit from cardiac pacing.
3. The leadless pacemaker may be deployed at a target site so as to be oriented in the same direction as the extension of a blood vessel so that the pacing and sensing electrodes are positioned at different heights, and is thus applicable to patients in need of pacing and sensing at different height in a blood vessel. It may also be deployed so as to be oriented in the cross sectional direction of a blood vessel so that the pacing and sensing electrodes are positioned at the same height in the vessel wall, and is thus applicable to patients in need of pacing and sensing at the same height in a blood vessel.
4. Since the curved tubular housing of the leadless pacemaker is made of an elastically deformable material, when deployed at a target site, it is elastically deformable in conformity with the geometry of a space available at the site. As a result, the leadless pacemaker can sufficiently interact with, and is thus securely fixed to, body tissue around the site without unintentional dislodgement or other issues.
5. The ability of the fixation mechanism in the leadless pacemaker to unfold and fold with respect to the curved tubular housing allows easier implantation, fixation and retrieval of the leadless pacemaker.
6. The leadless pacemaker system incorporating the leadless pacemaker can fix the leadless pacemaker not only in an atrium or a ventricle but also on the inner wall of the superior or inferior vena cava. Moreover, with the aid of a bendable delivery sheath, the leadless pacemaker can be even fixed to the interatrial or interventricular septum. Therefore, it can be used for pacing from any of those regions and is applicable to a wide range of patients.

### BRIEF DESCRIPTION OF THE DRAWINGS

Fig. 1 is a schematic stereoscopic view of a leadless pacemaker according to an embodiment of the present invention.
Fig. 2 is a schematic top view of the leadless pacemaker of Fig. 1.
Fig. 3 is a structural schematic of a leading end portion of the leadless pacemaker of Fig. 1.
Fig. 4 is a structural schematic of the leadless pacemaker according to another embodiment.
Fig. 5 is a structural schematic of the leadless pacemaker according to yet another embodiment.
Fig. 6 is a structural schematic of the leadless pacemaker according to a further embodiment.
Fig. 7 is a structural schematic of a delivery member in a leadless pacemaker system according to an embodiment of the present invention.
Fig. 8 is a structural schematic of the delivery member being assembled with the leadless pacemaker in the leadless pacemaker system according to an embodiment of the present invention.
Fig. 9 is a structural schematic of the leadless pacemaker being loaded in the delivery member in the leadless pacemaker system according to an embodiment of the present invention.
Fig. 10 is a structural schematic of a guide member in the leadless pacemaker system according to an embodiment of the present invention.
Fig. 11 is a structural schematic of the guide member in the leadless pacemaker system being delivered into the heart according to an embodiment of the present invention.
Fig. 12 is a structural schematic of the leadless pacemaker in the leadless pacemaker system being deployed in the superior vena cava according to an embodiment of the present invention.

In these figures,
11-pacing electrode; 12-first curved tubular segment of a curved tubular housing; 121-leading end of the curved tubular housing; 13-sensing electrode; 14-coupling mechanism; 15-sharp-pointed hook; 16-hook receiving recess; 17-second curved tubular segment of the curved tubular housing; 18-sharp tip; 21-delivery sheath; 22-rotating sheath; 221-blind hole; 31-expanding sheath; 32-guide sheath; 40-heart; 41-inferior vena cava; 42-right atrium; 43-superior vena cava; D-gap.

### DETAILED DESCRIPTION

Objectives and features of the present invention will become more readily apparent from the following detailed description of the subject matter of the present invention, which is to be read in connection with the accompanying drawings. However, the present invention may also be embodied in other forms and is in no way limited to the following embodiments. It is to be noted that the figures are provided in a very simplified form not necessarily drawn to exact scale for the only purpose of helping to explain the disclosed embodiments in a more convenient and clearer way. It is to be also noted that a "leading end" of any component described herein refers to the end of the component that first enters a patient's body when the component is being implanted into the patient's body. Correspondingly, a "trailing end" of any component described herein refers to the end of the component that last enters or remains outside a patient's body when the component is being implanted into the patient's body. An "axial direction" of a curved tubular housing described herein refers to the direction in which the curved tubular housing extends, while a "radial direction" of the curved tubular housing refers to a direction perpendicular to the direction in which the curved tubular housing extends. When the curved tubular housing has a circular cross-sectional shape, the radial direction of the curved tubular housing refers to its diametric direction, while a "circumferential direction" of the curved tubular housing refers to the direction that follows its circumference at any axial position.

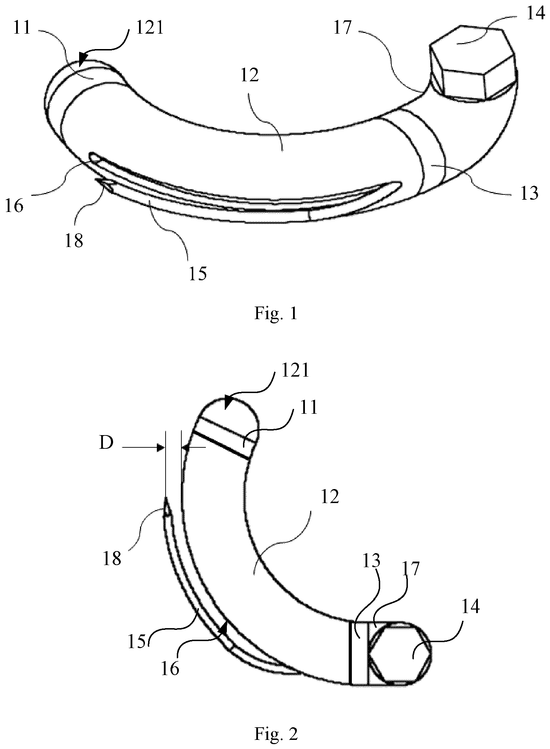

Referring to Figs. 1 to 3, a leadless pacemaker according to an embodiment of the present invention includes a pacemaker body, a fixation mechanism, a sensing electrode 11, a pacing electrode 13 and a coupling mechanism 14. The pacemaker body includes an elastically deformable curved tubular housing and electronic components (not shown) arranged inside the curved tubular housing. The electronic components may be various electronic components required for operation of the leadless pacemaker, such as a control module, a wireless communication electronic module, a battery module and a storage device. The control module may include an impulse generator and a processor. The impulse generator may be configured to generate pacing signals in the form of impulses, and the processor is configured to control delivery of the pacing signals and to perform other functions of the leadless pacemaker. The battery module may be configured to supply power to the control module, the storage device and other components. The storage device may be a register or the like for storing data, related parameters and the like. The wireless communication electronic module may be configured for bidirectional wireless communication with an external device (e.g., a programmer used by a clinician or another user in a medical facility, a household monitor or handheld device in the patient's premise, etc.). The external device may be used to configure and retrieve parameters of the leadless pacemaker (including parameters characterizing the operating status of the leadless pacemaker and the patient's cardiac parameters sensed by the leadless pacemaker).

The curved tubular housing is a hermetic housing made of a biocompatible, lightweight, highly elastically deformable material. Examples of this material include shape memory alloys such as nickel-titanium alloys and other highly elastic polymeric materials. The material enables the housing to be lightweight as much as possible and sufficiently elastic and resilient to deform in compliance with a spatial geometry around a site where the pacemaker is implanted. As a result, better fixation can be achieved to allow the pacing and sensing electrodes to more closely fit the surrounding body tissue at the implantation site to provide better pacing and sensing performance. The curved tubular housing may include a first curved tubular segment 12 and a second curved tubular segment 17. A leading end of the second curved tubular segment 17 may be coupled to a trailing end of the first curved tubular segment 12, and a trailing end of the second curved tubular segment 17 may be elevated over the first curved tubular segment 12. A leading end of the first curved tubular segment 12 may form a leading end of the curved tubular housing, and the trailing end of the second curved tubular segment 17 may form a trailing end of the curved tubular housing. With this design, the second curved tubular segment 17 is able to deflect and steer the first curved tubular segment 12 in a configuration where the first curved tubular segment 12 has deformed under the action of an external force to guide it smoothly back into a delivery system, thus achieving smooth delivery and retrieval of the pacemaker body.

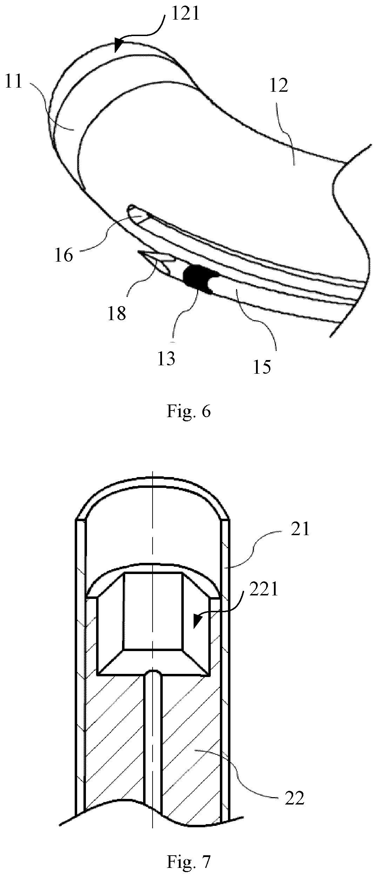

In this embodiment, both the first and second curved tubular segments 12, 17 are arc-shaped sections, and the first and second curved tubular segments 12, 17 each have circular openings at both ends (i.e., having a circular cross section along the radial direction that is perpendicular to the axial direction, as shown in Fig. 1). With this arrangement, the first curved tubular segment 12 transitions smoothly to the second curved tubular segment 17, thus additionally facilitating smooth delivery and retrieval of the pacemaker body. The first curved tubular segment 12 may have a length greater than a length of the second curved tubular segment 17. As the second curved tubular segment 17 elevated at the trailing end over the first curved tubular segment 12 is able to deflect and steer the first curved tubular segment 12 in a configuration where the first curved tubular segment 12 has deformed under the action of an external force to guide it smoothly through a delivery sheath 21 as shown in Figs. 7 to 9, the second curved tubular segment 17 determines whether smooth delivery and retrieval of the pacemaker body is achievable. When the trailing end of the second curved tubular segment 17 is elevated over the first curved tubular segment 12 at an angle of 85° to 95°, e.g., 90°, the second curved tubular segment 17 allows the first curved tubular segment 12 to be so released at a target site as to be oriented horizontally or almost horizontally. Moreover, the leading end 121 of the first curved tubular segment 12 may define a spherical tip, which can facilitate advancement of the pacemaker body when it is being released by the delivery sheath 21 as shown in Figs. 7 to 9 while avoiding the pacemaker body from piercing and damaging myocardial or vascular tissue against which it is brought into abutment during its release and extension at the target site or even perforating the myocardium or blood vessel.

The sensing electrode 11 may be disposed at the leading end 121 of the first curved tubular segment 12, and the pacing electrode 13 may be disposed at the trailing end of the first curved tubular segment 12 or at the trailing end of the second curved tubular segment 17. Moreover, both the sensing electrode 11 and pacing electrode 13 may be disposed circumferentially around the first curved tubular segment 12. The sensing electrode 11 may extend along an entire circumference, or part thereof, of an outer wall of the first curved tubular segment 12 around the leading end 121. The pacing electrode 13 may extend along an entire circumference, or part thereof, of the outer wall of the first curved tubular segment 12 around the trailing end or of an outer wall of the second curved tubular segment 17 around the trailing end. This co-radial design of the sensing and pacing electrodes creates a greatly increased chance of pacing a patient's heart implanted with the leadless pacemaker with ideal electrical parameters.

Since the first and second curved tubular segments 12, 17 are both arc-shaped, a length of the curved tubular housing (i.e., the sum of arc lengths of the first and second curved tubular segments 12, 17) is greater than a straight-line distance between the leading end of the first curved tubular segment 12 and the trailing end of the second curved tubular segment 17. Therefore, the curved-shaped leadless pacemaker according to this embodiment can be deployed at a site with a limited space (dimensioned less than the length of the curved tubular housing) and is thus applicable to patients with ideal pacing sites having limited spaces.

Additionally, since the first and second curved tubular segments 12, 17 of the curved tubular housing according to this embodiment are both arc-shaped, with the co-radial pacing electrode 13 and sensing electrode 11 being spaced from each other along an axis of the first curved tubular segment 12 and with the second curved tubular segment 17 being elevated over the first curved tubular segment 12 at an angle of 90°, when deployed on a curved surface such as the superior vena cava wall, the leadless pacemaker according to this embodiment can more closely fit the tissue with sufficient contact and substantially no clearance left. As a result, improved pacing and sensing performance can be achieved with the pacing electrode operating normally to deliver pacing signals and the sensing electrode operating normally to sense signals from the tissue. More importantly, the pacemaker body may be oriented to extend in a circumferential direction of the superior vena cava wall so that the pacing electrode 13 and sensing electrode 11 are spaced from each other in the same direction at the same height within the blood vessel. In this way, the leadless pacemaker is made applicable to patients for whom pacing with ideal electrical parameters would be achieved when the leadless pacemaker is deployed on a curved surface such as the superior vena cava wall. Similarly, the leadless pacemaker according to this embodiment may also be deployed on other curved surfaces. In this way, it is applicable to a substantially enlarged scope of patients who may benefit from cardiac pacing. All of these are impossible for conventional straight elongate leadless pacemakers because when deployed in the superior vena cava, the pacemaker body of such a leadless pacemaker has to be oriented in the same direction as the extension of the superior vena cava. Consequently, the pacing and sensing electrodes are located at different heights in the superior vena cava, making the pacemaker unsuitable for use in patients in need of pacing electrode and sensing electrode at the same height in the blood vessel. Additionally, when the conventional straight elongate leadless pacemaker is deployed on a curved surface, there will be one or more clearances left between it and the tissue surface because sufficient contact is unachievable therebetween. As a result, the pacing electrode may not be able to operate normally to deliver pacing signals, and/or the sensing electrode may not be able to operate normally to sense signals from the tissue. Therefore, the conventional straight elongate leadless pacemakers cannot be deployed on curved surfaces and are thus not able to pacing the hearts of patients in need thereof with ideal electrical parameters.

In this embodiment, the fixation mechanism is a hook-based fixation mechanism including at least one sharp-pointed hook 15. Each sharp-pointed hook 15 may assume unfolded and folded positions relative to the first curved tubular segment 12. Specifically, at least one hook receiving recess 16 each for receiving a corresponding respective one of the respective sharp-pointed hook(s) 15 may be provided in a convexly curved outer surface portion of the first curved tubular segment 12 in the curved tubular housing. A trailing end of each sharp-pointed hook 15 may be fixed, e.g., welded or pressed, in the corresponding hook receiving recess 16, and a leading end of the sharp-pointed hook 15 may extend toward the leading end 121 of the first curved tubular segment 12. This hook-based fixation mechanism is simple in structure, easy to be fabricated and has a small footprint. Moreover, it facilitates the transfer of a torque applied by an operator's rotating action performed on the delivery sheath in the delivery system to each sharp-pointed hook 15 so that the sharp-pointed hook 15 can penetrate and anchor into tissue, thus achieving the fixation of the leadless pacemaker. In addition to a small footprint, the hook-based fixation mechanism acts essentially at the leading end(s) of the sharp-pointed hook(s). Therefore, it can fix the leadless pacemaker not only in an atrium or ventricle of the heart but also onto the inner wall of the superior or inferior vena cava. With the aid of a bendable delivery sheath, it can even fix the leadless pacemaker to the interatrial or interventricular septum or a similar site.

Each sharp-pointed hook 15 may be formed of a biocompatible, lightweight, highly elastic material such as a shape memory metallic material such as a nickel-titanium alloy. In this way, it is lightweight as much as possible, sufficiently elastic and resilient. Moreover, it can provide sufficient fixation while almost not increasing the weight of the leadless pacemaker, reducing the pushing or pulling force applied by the leadless pacemaker to surrounding tissue at an implantation site. Each sharp-pointed hook 15 may be generally in the form of a wire, a rod or a tab. Moreover, it may extend over, or even be wound on, the outer circumferential wall of the first curved tubular segment 12 over the total (arc) length of the first curved tubular segment 12. In this way, each sharp-pointed hook 15 may closely fit on the curved tubular housing when in the folded position, facilitating the delivery of the leadless pacemaker. In addition, after it is released at a target implantation site, it can assume the unfolded position where it anchors to body tissue, thereby providing sufficient support to the pacemaker body and ensuring its secure fixation. Each hook receiving recess 16 may be dimensioned both widthwise and lengthwise to completely receive the corresponding sharp-pointed hook 15 so that the sharp-pointed hook 15 can be received in the corresponding hook receiving recess 16. This facilitates smooth delivery of the leadless pacemaker by the delivery system.

When in a rest state, the leading end of each sharp-pointed hook 15 may be spaced from the outer circumference of the first curved tubular segment 12 by a gap D in the range of 0.1 mm to 15 mm. When the sharp-pointed hook 15 is relieved from any external force as a result of release of the leadless pacemaker, the gap D allows the leading end thereof to extend by its own resilience to pierce body tissue around the implantation site. The gap D may be determined according to conditions of a site where the leadless pacemaker is to be deployed. In other words, in order to address various accommodation spaces available at different sites, multiple options of the leadless pacemaker with different curved tubular housing lengths, volumes, curvatures and different sharp-pointed hook 15 sizes, volumes and gaps relative to the outer wall of the curved tubular housing may be provided.

Under the action of an external force acting on the leading end of each sharp-pointed hook 15, the sharp-pointed hook 15 may be received within the corresponding hook receiving recess 16 along the trajectory of the hook receiving recess 16. During delivery of the leadless pacemaker by the delivery sheath 21 as shown in Figs. 7 to 9, a torque may be applied to drive the delivery sheath 21 as shown in Figs. 7 to 9 to rotate to cause a sharp tip 18 at the leading end of each sharp-pointed hook 15 to pierce and anchor into tissue. In this way, the leadless pacemaker may be fixed within an atrium or ventricle of the heart, or onto the inner wall of the superior or inferior vena cava. When the delivery sheath used to deliver the leadless pacemaker is flexibly bendable, the leadless pacemaker can be fixed even onto the atrial or ventricular septum by virtue of the sharp-pointed hook(s) 15. Arranging the sharp-pointed hook(s) 15 on a convexly curved outer surface portion (i.e., an outer surface portion on the external side) of the first curved tubular segment 12 can take advantage of a squeezing force between the outer surface of the first curved tubular segment 12 and tissue around the implantation site to enhance reliability of the fixation provided by the sharp-pointed hook(s) 15 and prevent its/their dislodgement. Of course, if the curved tubular housing is sufficiently long, the sharp-pointed hook(s) 15 may be designed to be long enough, so that the sharp-pointed hook(s) 15 may be alternatively arranged on a concavely curved outer surface portion of the curved tubular housing.

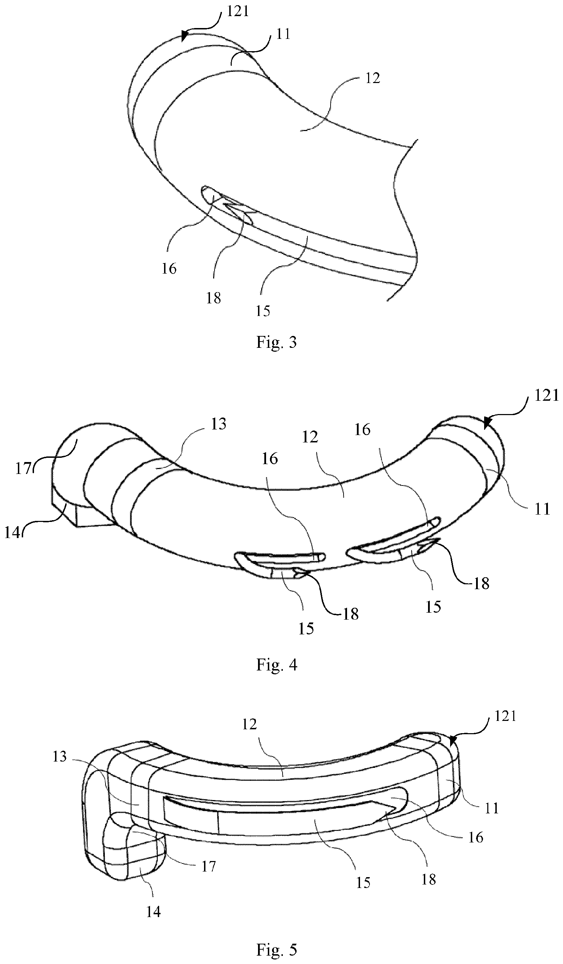

Referring to Fig. 4, the fixation mechanism preferably includes two or more sharp-pointed hooks 15, each sharp-pointed hook 15 is arranged at different locations of the first curved tubular segment 12. This allows the leadless pacemaker to be more firmly fixed at multiple points at the implantation site. In other words, when the leadless pacemaker is released at a target site, the sharp-pointed hooks 15 will penetrate and anchor to surrounding body tissue (e.g., the right ventricular apex) at the target site, providing the leadless pacemaker with multiple fixation points. Additionally, all the sharp-pointed hooks 15 may have equal lengths and gaps D. With this arrangement, after the leadless pacemaker is released, the sharp-pointed hooks 15 will exert equal forces, making the fixation of the leadless pacemaker more secure and firm without unwanted tilting. Of course, in other embodiments of the present invention, in order for secure and firm fixation of the leadless pacemaker to be achieved, the sharp-pointed hooks 15 may also have unequal lengths and/or gaps D in adaptation to a particular curved surface at the target site. It would be recognized that the hook-based fixation mechanism 14 is a structurally simple low-cost mechanism that allows easy release and fixation of the leadless pacemaker.

Referring to Fig. 5, more preferably, each sharp-pointed hook 15 may have a rectangular cross section along the radial direction of the curved tubular housing (referred to as the cross section of the sharp-pointed hook 15 hereinafter for short), which allows the sharp-pointed hook, when released at an implantation site and assuming an extended configuration, to have an increased contact area with tissue or a blood vessel wall around the implantation site. Alternatively, the curved tubular housing may have a rectangular cross section along the radial direction thereof (referred to as the cross section of the curved tubular housing hereinafter for short). Likewise, this can result in an increased contact area between the curved tubular housing and the tissue or blood vessel wall around the implantation site. This rectangular cross-sectional design allows the pacing electrode 13 and sensing electrode 14 to each have a larger contact area with the tissue around the implantation site, which enables stable pacing with optimized electrical parameters.

Of course, in other embodiments of the present invention, the first and second curved tubular segments 12, 17 may be otherwise curved than arc-shaped. For example, they may be wave-shaped, ς-shaped or S-shaped. In addition, the openings of the first and second curved tubular segments 12, 17 may have another shape than circular. For example, the shape of the openings may alternatively be elliptical or polygonal (e.g., rectangular, as shown in Fig. 5), defined by a closed wavy line, or another shape. The sharp-pointed hooks 15 may have another cross-sectional shape than circular or rectangular, such as elliptical, triangular or otherwise polygonal. As long as the leadless pacemaker is allowed to perform its intended pacing function, have a reduced size enabling it to move forward in an intravenous delivery sheath into the heart or a blood vessel, and be deployed at a target site so that the curved tubular housing and the sharp-pointed hooks fit body tissue around the site as closely as possible, all such alternative embodiments are considered to fall within the scope of the present invention. For example, some patients may have a defect in the heart or in a blood vessel. If the leadless pacemaker is to be deployed at such a defective site, in order to enable the curved tubular housing to fit body tissue around the site as closely as possible with an increased contact area therewith, the first and second curved tubular segments 12, 17 may be selected as wave-, ς- or S-shaped segments having a cross sectional shape that is polygonal or defined by a closed wavy line. Additionally, the sharp-pointed hooks 15 may have an overall shape adapted to that of the outer wall of the curved tubular housing so that the sharp-pointed hooks are folded to complement the outer surface of the curved tubular housing during delivery of the leadless pacemaker and fit the body tissue around the defective site as closely as possible after the leadless pacemaker is deployed at the site. In this way, desirable sensing and pacing performance of the leadless pacemaker can be expected. Preferably, the cross-sectional shape of the curved tubular housing is similar to that of the sharp-pointed hooks 15 so that when folded in place, the sharp-pointed hooks 15 can complement the outer surface of the curved tubular housing.

It is to be noted that, in other embodiments of the present invention, the hook receiving recesses 16 may be omitted from the outer wall of the curved tubular housing, and fasteners may be instead arranged on the curved tubular housing. In this case, when the sharp-pointed hooks 15 are folded in place, they will be fastened by the fasteners so that they are brought into close contact with the outer wall of the curved tubular housing. That is, the sharp-pointed hooks 15 are elevated over the outer wall of the curved tubular housing. In this case, similar beneficial effects can be achieved. It would be appreciated that, in other embodiments of the present invention, the fixation mechanism may adopt any other suitable fixation approach, as long as it is allowed to reliably fix the leadless pacemaker at a target site in a patient's body while being free of serious issues like insecure fixation, atrial perforation, inferior pacing performance, inferior sensing performance, etc. For example, in some embodiments, the fixation mechanism of the leadless pacemaker is designed to abut against rather than penetrate body tissue. In this case, similar secure fixation can be achieved by a squeezing force between the leadless pacemaker and the body tissue, ensuring good pacing and sensing performance.

Referring to Figs. 1 to 3, the coupling mechanism 14 is arranged at the trailing end of the second curved tubular segment 17, the coupling mechanism 14 is configured for coupling to a delivery member for delivering the leadless pacemaker (e.g., the rotating sheath 22 shown in Figs. 7 to 9). The coupling mechanism 14 may be integrally formed with the second curved tubular segment 17, or secured by welding or threading to the second curved tubular segment 17 at the trailing end thereof. In this embodiment, the coupling mechanism 14 is a polygonal (e.g., hexagonal) prism for mating with a correspondingly polygonal blind hole (indicated at 221 in Fig. 7) formed in the delivery member for delivering the leadless pacemaker. The mating between the polygonal prism and the polygonal blind hole allows easy attachment and detachment of the leadless pacemaker to and from the delivery member, in particular, simply by rotating the rotating sheath less than one turn, thus reducing the risk of unsuccessful implantation. In other embodiments of the present invention, the coupling mechanism 14 may be a nut with a blind hole for mating with a bolt provided on the delivery member for delivering the leadless pacemaker. Alternatively, the coupling mechanism 14 may be a threaded bolt for mating with a nut with a threaded hole provided on the delivery member for delivering the leadless pacemaker.

Although the sensing and pacing electrodes 11, 13 have been described in the above embodiments as being both provided on the curved tubular housing, the present invention is not so limited. For example, with reference with Fig. 6, when only one sharp-pointed hook 15 is provided, the sensing electrode 11 or the pacing electrode 13 may be provided on the sharp-pointed hook 15. When two or more sharp-pointed hooks 15 are provided, the sensing electrode 11 and the pacing electrode 13 may be provided on different ones of the sharp-pointed hooks 15. Each of the sensing and pacing electrodes may be adhered to, embedded in, or otherwise provided on one of the sharp-pointed hook(s) 15. As another example, when fabricated from a highly elastic shape memory alloy, the sharp-pointed hook(s) 15 will be electrically conductive. In this case, each sharp-pointed hook 15 may be used as the sensing electrode 11 or the pacing electrode 13 and coupled via an associated circuit to an electronic component inside the curved tubular housing to perform the pacing or sensing function. Using the sharp-pointed hook(s) 15 as electrode(s) dispenses with the need to fabricate separate electrode(s) and allows a simpler structure of the leadless pacemaker.

It is to be noted that although the second curved tubular segment 17 has been described in the above embodiments as being elevated over the first curved tubular segment 12 at an angle of 85°-95° so that the leadless pacemaker can be deployed in a horizontal orientation with the pacing and sensing electrodes being located at the same height within the superior vena cava, the present invention is not so limited. In another embodiment of the present invention, a tilt angle of the rotating sheath 22 may be controlled so that the leadless pacemaker is deployed so as to extend in the same direction as the superior vena cava with the pacing and sensing electrodes being located at different heights in the superior vena cava. In yet another embodiment of the present invention, the second curved tubular segment 17 may be designed to be elevated over the first curved tubular segment 12 at an angle of 45° or less or omitted. In this case, the leadless pacemaker may be deployed in the same orientation as the straight elongate leadless pacemakers, i.e., it extends in the same direction as the superior vena cava with the pacing and sensing electrodes being situated at different heights in the superior vena cava, to address the needs of similar patients.

In summary, the leadless pacemaker of the present invention has improved pacing or sensing performance because the housing of the pacemaker body is designed as a curved tubular structure capable of more closely fitting myocardial or vascular tissue with the same curved geometry. Moreover, the housing of the pacemaker body is made of an elastically deformable material, making the leadless pacemaker deployable not only in the superior vena cava but also at other sites with curved surfaces and thus making it applicable to a substantially enlarged scope of patients who may benefit from cardiac pacing. Further, the pacing electrode and/or the sensing electrode is/are co-radial with respect to (i.e., arranged on the same circumference of) the curved tubular housing of the pacemaker body. In this way, the leadless pacemaker may be deployed, for example, in the superior vena cava or at an endocardial site, so that the pacemaker body extends along a circumference of the blood vessel wall or myocardial wall. As a result, the pacing electrode and/or the sensing electrode is/are located on the same circumference and hence at the same height with respect to the blood vessel wall or myocardial wall. In this way, the leadless pacemaker is made applicable to patients for whom pacing with ideal electrical parameters would be attained when the leadless pacemaker is deployed with the pacing and sensing electrodes located at the same height with respect to a blood vessel wall or a myocardial wall.

Referring to Figs. 1 to 12, in an embodiment of the present invention, there is also provided a leadless pacemaker system including a delivery member, a guide member and the leadless pacemaker of the present invention as defined above. The delivery member is detachably coupled to the trailing end of the pacemaker body of the leadless pacemaker and configured to deliver and tune the leadless pacemaker to an optimal pacing site with the aid of the guide member. The guide member is configured to set up a path for the delivery member so that the delivery member reaches the pacing site after passing though the guide member. The delivery and guide member can be used in cooperation with each other in a surgical procedure to deliver the leadless pacemaker to an endocardial or endovascular target site, followed by its fixation thereat, avoiding the side effects arising from the use of a traditional pacemaker with electrode leads. During implantation of the leadless pacemaker, the guide member is able to guide the delivery member that is loaded therein with the leadless pacemaker to the vicinity of a target site in a patient's body, and the delivery member is able to help adjust the leadless pacemaker in position, facilitating the operator's easy identification of an ideal pacing site for the leadless pacemaker, which is helpful in improving the leadless pacemaker's pacing performance.

Reference may be made to the above description for details in the structure of the leadless pacemaker in the leadless pacemaker system according to this embodiment.

Referring to Figs. 7, 8 and 9, in this embodiment, the delivery member includes a delivery sheath 21 and a rotating sheath 22. The delivery sheath 21 is a circular tubular structure, and the rotating sheath 22 is provided at a leading end thereof with a polygonal blind hole 221 capable of mating with the coupling mechanism 14 of the leadless pacemaker. The polygonal blind hole 221 is complementary in shape to the coupling mechanism 14, allowing attachment and detachment of the leadless pacemaker to and from the rotating sheath 22. In other words, the rotating sheath 22 is detachably coupled at the leading end thereof to the trailing end of the second curved tubular segment 17 in the leadless pacemaker. The delivery sheath 21 is movable axially (i.e., forth and back relative to the rotating sheath 22) and rotatable circumferentially (relative to the rotating sheath 22).

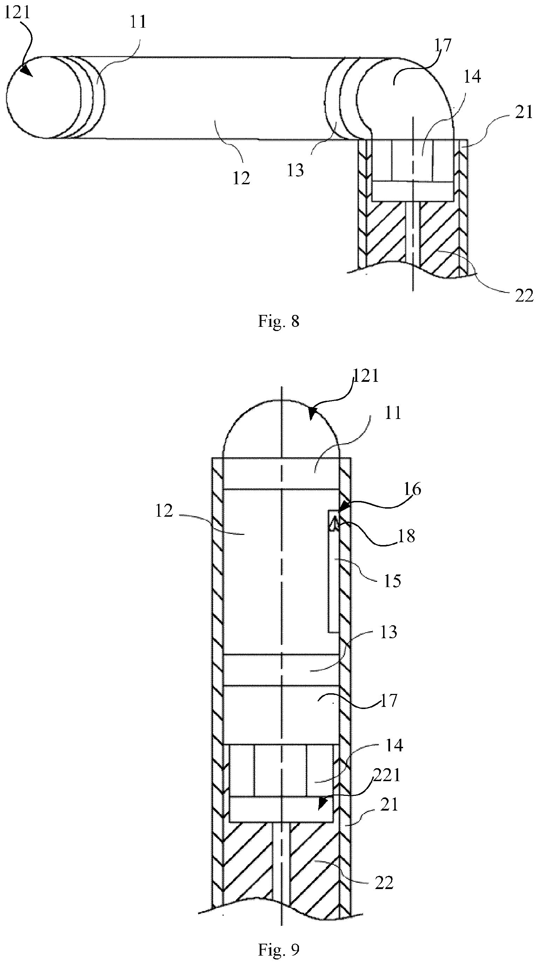

Assembly of the delivery member with the leadless pacemaker begins with inserting the rotating sheath 22 into the delivery sheath 21, followed by plugging the coupling mechanism 14 at the trailing end of the leadless pacemaker, from a leading end of the delivery sheath 21, into the polygonal blind hole 221 at the leading end of the rotating sheath 22, resulting in the second curved tubular segment 17 of the curved tubular housing of the leadless pacemaker being received in a central bore of the delivery sheath 21. Subsequently, with the rotating sheath 22 being held still, the delivery sheath 21 is pushed in an axial direction of the rotating sheath 22 toward the leading end 121 of the curved tubular housing so that the curved tubular housing elastically deforms and is entirely received in the central bore of the delivery sheath 21, concurrently with each sharp-pointed hook 15 being retracted and folded into the corresponding complementary hook receiving recess 16. As shown in Fig. 9, the leadless pacemaker deforms to conform to the shape of the central bore of the delivery sheath 21.

Preferably, the delivery sheath 22 is a curved sheath with a degree of curvature adjustable within the range of 0°-180°. This curvature adjustability can adapt the delivery sheath 22 to various spatial geometries within the heart and blood vessels, thus helping deploy the leadless pacemaker at any site of the atria, ventricles, inner wall of the superior vena cava, inner wall of the inferior vena cava and atrial and ventricular septa to allow it to perform the pacing function there.

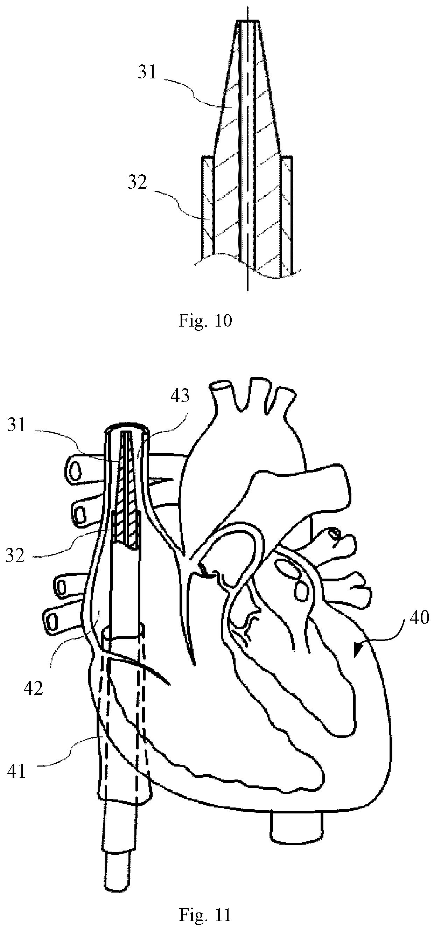

Referring to Fig. 10, the guide member includes an expanding sheath 31 and a guide sheath 32. The expanding sheath 31 can be received in the guide sheath 32 so as to be movable in the guide sheath 32 axially. The expanding sheath 31 has a tapered leading end. The expanding sheath 31 can be received in the guide sheath 32 so that the tapered leading end of the expanding sheath 31 protrudes out of the guide sheath 32. Holders (not shown) may be provided at trailing ends respectively of the expanding sheath 31 and of the guide sheath 32. When the tapered leading end of the expanding sheath 31 protrudes out of the guide sheath 32 from a leading end thereof, the holder at the trailing end of the expanding sheath 31 may abut against, and be optionally coupled by threading or arresting to, the holder at the trailing end of the guide sheath 32. The guide sheath 32 is able to move axially together with the expanding sheath 31, and the expanding sheath 31, when advancing within a blood vessel in a patient's body, is able to radially expand the blood vessel to allow smooth advancement of the guide sheath 32 therein. The expanding sheath 31 defines a central bore along its central axis, through which a guidewire can be inserted. In order to deploy the leadless pacemaker, the guidewire may be first inserted through the central bore and secured at a leading end thereof to tissue around a target site (e.g., in the superior vena cava), followed by inserting the expanding sheath 31 in the guide sheath 32 and inserting the guidewire through both the expanding sheath 31 and the guide sheath 32. In this way, the guide sheath 32 may be guided to a desired location by moving together with the expanding sheath 31 over the guidewire.

In this embodiment, the leadless pacemaker system further includes an external electronic control device, which is connected by a wireless connection to, and can communicate in a bilateral manner with, the leadless pacemaker to configure and retrieve parameters of the leadless pacemaker (including parameters characterizing the operating status of the leadless pacemaker and the patient's cardiac parameters sensed by the leadless pacemaker).

Referring to Figs. 1 to 12, in an embodiment, there is also provided a method of using the leadless pacing system as defined above, as detailed below.

At first, referring to Fig. 8, prior to implantation, the rotating sheath 22 is inserted in the delivery sheath 21, and the coupling mechanism 14 at the trailing end of the pacemaker body of the leadless pacemaker is then inserted into the delivery sheath 21 and the coupling mechanism 14 is mated with the polygonal blind hole 221 at the leading end of the rotating sheath 22, resulting in the second curved tubular segment 17 of the curved tubular housing of the pacemaker body being received in the central bore of the delivery sheath 21. Subsequently, referring to Fig. 9, with the rotating sheath 22 being held stationary, the delivery sheath 21 is pushed to move axially with respect to the rotating sheath 22 toward the leading end of the pacemaker body (i.e., the leading end 121 of the first curved tubular segment 12 of the curved tubular housing) so that the curved tubular housing elastically deforms to be entirely received in the central bore of the delivery sheath 21, concurrently with each sharp-pointed hook 15 being retracted and folded into the corresponding complementary hook receiving recess 16.

Afterward, referring to Fig. 11, the guidewire (not shown) is then introduced into a patient's body via a femoral vein (not shown) and advanced through the inferior vena cava 41 and the right atrium 42 of the heart 40 toward the superior vena cava 43. The expanding sheath 31 is then inserted into the guide sheath 32 so that the leading end of the expanding sheath 31 protrudes out of the guide sheath 32 from the leading end thereof, with the holder (not shown) at the trailing end of the expanding sheath 31 abutting against the holder (not shown) at the trailing end of the guide sheath 32. The two trailing holders are then fastened to each other with fasteners provided thereon. The expanding sheath 31 is then disposed over the guidewire and slowly advanced thereon while being monitored using X-way so that the expanding sheath 31, together with guide sheath 32, is introduced via the femoral vein and advanced through the inferior vena cava 41 and the right atrium 42 to a desired location in the superior vena cava 43. Thereafter, the holders of the guide sheath 32 and the expanding sheath 31 are unfastened, and the expanding sheath 31 and the guidewire are withdrawn, with the guide sheath 32 being retained therein.

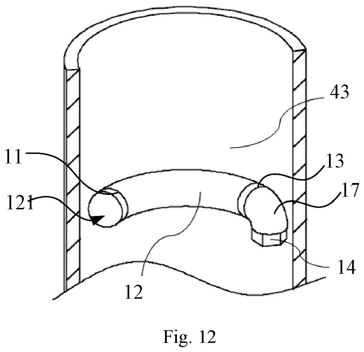

After that, with continued reference to Figs. 9 and 11, the leadless pacemaker and a leading end of the delivery sheath 21 in the configuration shown in Fig. 9 are together inserted into the guide sheath 32 from the trailing end thereof. With the guide sheath 32 being held still, the delivery sheath 21 and the rotating sheath 22 are together pushed forward axially in the guide sheath 32 until the leading end 121 of the curved tubular housing is located at the leading end of the guide sheath 32. Then, with the rotating sheath 22 and the guide sheath 32 being held stationary, the delivery sheath 21 is retracted along the axial direction of the guide sheath 32 until the rotating sheath 22 returns to the position as shown in Figs. 7 and 8. As a result, the curved tubular housing is released from the delivery sheath 21 and regains its curved shape as shown in Fig. 8. At this point, the trailing end of the rotating sheath 22 is manipulated to bring the pacing and sensing electrodes 13, 11 into contact with the wall of the superior vena cava 43, and an external device is then used to determine whether electrical parameters of the pacing and sensing electrodes 13, 11 are satisfactory. If it is found that pacing and sensing electrical parameters are unsatisfactory due to non-ideal pacing and sensing locations, the delivery sheath 21 may be moved axially to retrieve the leadless pacemaker therein, followed by positional adjustments made to the leadless pacemaker through axial translational movement of the delivery sheath 21 and rotational movement of the rotating sheath 22. When a desirable new site is reached, the delivery sheath 21 is again retracted to release the leadless pacemaker, and the external device is again used to obtain measurements of pacing and sensing electrical parameters from the new endocardial locations of the sensing and pacing electrodes. This process is repeated until desired or ideal electrical parameter measurements are obtained. When this happens, the trailing end of the rotating sheath 22 may be turned to drive both the sharp-pointed hooks 15 and the curved tubular housing to rotate so that the sharp tips 18 of the sharp-pointed hooks 15 pierce and anchor into the inner wall of the superior vena cava 43, as shown in Fig. 12, thereby securely fixing the leadless pacemaker to the ideal pacing site.

With continued reference to Fig. 12, at the end of the implantation process, the rotating sheath 22 is decoupled from the coupling mechanism 14, and all the other components than the leadless pacemaker, including the delivery sheath 21, the rotating sheath 22 and the guide sheath 32, are then withdrawn from the patient's body, with the leadless pacemaker remaining fixed in the superior vena cava 43.

In the same way as described in the foregoing embodiment, the operator may alternatively implant the leadless pacemaker at other target sites on the atrial wall, the ventricular wall, the inner wall of the inferior vena cava and the interatrial and interventricular septa, and it is intended that all such alternative embodiments are also embraced in the scope of the present invention.

Furthermore, the leadless pacemaker in the leadless pacemaker system according to this embodiment may be used in combination with one or more other leadless pacemakers by virtue of wired or wireless connection(s) established therewith to achieve true leadless dual-chamber or triple-chamber pacing, allowing the advanced technology, leadless pacing, to benefit more patients.

In summary, the leadless pacemaker system of the present invention enables the leadless pacemaker to be implanted not only in the superior vena cava but also at other similar sites associated with curved surface geometries, thus making it applicable to a substantially enlarged scope of patients who may benefit from cardiac pacing.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A leadless pacemaker, comprising:
a pacemaker body comprising a curved tubular housing;
a fixation mechanism disposed on an outer wall of the curved tubular housing;
a sensing electrode disposed outside the curved tubular housing; and
a pacing electrode disposed outside the curved tubular housing so as to be spaced apart from the sensing electrode.

2. The leadless pacemaker of claim 1, wherein the sensing electrode is disposed on the curved tubular housing so as to extend in a circumferential direction of the curved tubular housing to surround the outer wall of the curved tubular housing, or the sensing electrode is disposed on the fixation mechanism.

3. The leadless pacemaker of claim 1, wherein the pacing electrode is disposed on the curved tubular housing so as to extend in a circumferential direction of the curved tubular housing to surround the outer wall of the curved tubular housing, or the pacing electrode is disposed on the fixation mechanism.

4. The leadless pacemaker of any one of claims 1 to 3, wherein when the leadless pacemaker is delivered to a target pacing site, the pacing electrode and the sensing electrode are positionable at a same height.

5. The leadless pacemaker of claim 1, wherein the fixation mechanism is able to assume an unfolded or folded configuration with respect to the curved tubular housing.

6. The leadless pacemaker of claim 5, wherein the fixation mechanism comprises at least one sharp-pointed hook having a trailing end secured to the curved tubular housing and a leading end extending toward a leading end of the curved tubular housing, the leading end of the sharp-pointed hook spaced from an outer circumference of the curved tubular housing by a gap when in a rest state, thereby defining the unfolded configuration with respect to the curved tubular housing, the leading end of the sharp-pointed hook retracted onto the curved tubular housing under the action of an external force applied thereto, thereby defining the folded configuration with respect to the curved tubular housing.

7. The leadless pacemaker of claim 6, wherein a hook receiving recess is provided in the outer wall of the curved tubular housing and configured to receive the at least one sharp-pointed hook so that the trailing end of each sharp-pointed hook is secured in the hook receiving recess and that each sharp-pointed hook is received, under the action of an external force applied to the leading end of the sharp-pointed hook, in the hook receiving recess along an entire length thereof.

8. The leadless pacemaker of claim 6 or 7, wherein each sharp-pointed hook is provided in the form of a wire, a rod or a tab.

9. The leadless pacemaker of claim 6 or 7, wherein the sharp-pointed hook is arranged on a convexly curved outer surface portion of the curved tubular housing.

10. The leadless pacemaker of claim 6 or 7, wherein the pacing electrode or the sensing electrode is disposed on one of the sharp-pointed hook(s), and wherein both the pacing electrode and the sensing electrode are electrically connected to an electronic component inside the curved tubular housing.

11. The leadless pacemaker of claim 1, wherein the curved tubular housing comprises a first curved tubular segment and a second curved tubular segment, the second curved tubular segment having a leading end coupled to a trailing end of the first curved tubular segment, the second curved tubular segment having a trailing end elevated over the first curved tubular segment, the first curved tubular segment having a leading end forming a leading end of the curved tubular housing, the trailing end of the second curved tubular segment forming a trailing end of the curved tubular housing.

12. The leadless pacemaker of claim 11, wherein the first curved tubular segment is arc-shaped or wave-shaped, the second curved tubular segment is arc-shaped or wave-shaped, and a length of the first curved tubular segment is greater than a length of the second curved tubular segment.

13. The leadless pacemaker of claim 1, wherein the curved tubular housing has a cross section along its radial direction, which is polygonal or defined by a closed wavy line.

14. The leadless pacemaker of claim 1, wherein the curved tubular housing is made of an elastically deformable material.

15. The leadless pacemaker of claim 1, wherein the curved tubular housing is provided at a trailing end thereof with a coupling mechanism configured for coupling to a delivery member for delivering the leadless pacemaker.

16. A leadless pacemaker system, comprising:
the leadless pacemaker of any one of claims 1 to 15; and
a delivery member detachably coupled to the trailing end of the pacemaker body of the leadless pacemaker, the delivery member configured to deliver the leadless pacemaker to a target pacing site.

17. The leadless pacemaker system of claim 16, wherein the delivery member comprises a delivery sheath and a rotating sheath receivable in the delivery sheath, the delivery sheath axially movable and circumferentially rotatable relative to the rotating sheath, the rotating sheath having a leading end configured for detachable coupling to the trailing end of the pacemaker body of the leadless pacemaker.

18. The leadless pacemaker system of claim 17, wherein the delivery sheath is a curved sheath with a degree of curvature that is adjustable.
